# EUROPEAN PATENT SPECIFICATION

(11) **EP 1 589 869 B1**
(45) Date of publication and mention of the grant of the patent: **26.12.2007**
(21) Application number: 04708536.0
(22) Date of filing: 05.02.2004
(51) Int. Cl.: A61B 5/03, G06F 17/00

(54) **SYSTEM FOR PREVENTION OF WORK INJURIES**
SYSTEM ZUR VERHINDERUNG VON ARBEITSBEDINGTEN VERLETZUNGEN
SYSTEME DE PREVENTION D'ACCIDENTS DU TRAVAIL

(30) Priority: 06.02.2003 SE 0300338
(43) Date of publication of application: 02.11.2005
(73) Proprietor: Xperientia AB, 553 18 Jönköping (SE)
(72) Inventor: ROBERTSSON, Hans, S-554 48 Jönköping (SE)
(74) Representative: Willquist, Bo Lorentz
(86) International application number: PCT/SE2004/000158
(87) International publication number: WO 2004/069050

(56) References cited:
- GB-A- 2 343 767
- GB-A- 2 366 410
- US-A- 5 792 025
- US-A- 6 152 890
- US-B1- 6 296 408

## Description

The invention relates to a system for alleviating and preventing work injuries, primarily in an office environment but also, for example, in forestry work and timber handling, building and construction work and other work situations.

### Background of the invention

In office environments where there is prolonged use of computers, work-related stress injuries often occur. These lead, among other things, to pains in the elbows, shoulders, neck and back etc.

In order to alleviate these, increasing use is now being made of furniture that will allow flexible working positions. This furniture can help to reduce the stresses or make them more variable.

This involves, among other things, the use of height-adjustable desks and chairs with a wider range of possible adjustments.

The pains experienced, however, are often of an individual nature and difficult to analyse. For this reason, the flexibility of the furniture is seldom utilised to good effect. Computer work can be absorbing and focussed and before one realises it one has been sitting in a wrong position for too long doing persistent harm.

Document D1 discloses a computer usage management system for a safer working environment. The computer includes a user activity monitor for monitoring usage of the computer by a user. The user is also prompted to answer a questionnaire regarding seating position and posture. Feedback is given on the user's answers regarding if he is seating correctly or not.

It would therefore be desirable to supplement the adjustable furniture with a system that allows the individual to make effective use of its flexibility.

Similar problems and injuries can arise in sawing work when handling power saws, in building work when using working tools such as grinding and shaping machines and in other situations.

### Summary of the invention

The object of the present invention is to provide a system which is designed to map how an individual's work-related pain symptoms vary as a function of work activities and to make use of working positions, particularly on adjustable furniture. The system warns the individual when a situation is about to arise for which the mapping has shown that pain symptoms generally occur and it is therefore advisable to change working positions in order to prevent this.

The system comprises sensors on work equipment, particularly the items of adjustable furniture, which detect settings and adjustments of the tool or the relevant furniture, and sensors that detect computer-related activity such as keyboard and pointing device activities. The system also comprises the necessary hardware and software needed to record and store the status of the sensors as a function of the time and in appropriate cases also to record the nature of any pain symptoms experienced by the individual.

As data is thus gathered on the individual's behaviour and the resulting pain sensations, an analytical program (constructed as a neural network, for example), which is linked to the recording can identify correlations between the behaviour and pain symptoms. The individual can thereby be alerted and encouraged to alter his or her working position before it is too late. If such a warning and subsequent adjustment of the working position proves successful, the neural network can then further improve its preventive warning capability.

The system according to the invention has the characterising features specified in claim 1. Embodiments of the invention have the characterising features revealed by the other patent claims.

### Description

The invention will now be described on the basis of the following non-limitative examples of embodiment.

It is assumed that an individual has a work station comprising a height-adjustable desk, a computer with keyboard, display screen and pointing device, and also an office chair with adjustment facilities.

It is furthermore assumed that the height-adjustable desk is provided with a sensor which is capable of recording the height of the desk, and that a program on the computer records the keyboard activity and point device movements and what computer program the individual is working with.

The chair is also provided with sensors which record chair movements, the adjustment position of the chair and where appropriate also the seated posture of the individual. The latter can be accomplished, for example, by one or more load cells fitted to the chair. In a preferred embodiment of the invention the system also comprises means, sensors, located on the individual for recording stresses and/or movements and for transmitting this information and storing and processing it on the computer.

The sensors are appropriately equipped for wireless communication and provided with long-life battery and communicate sensor data to hardware and software which are suited to the purpose and linked to the computer.

On the computer there is also interactive software allowing the individual to record pain symptoms. This dialogue can either be activated by the individual himself when he feels pain or automatically activated when the program registers that the individual is changing or intends to change his working position. Communication with the individual can be achieved by means of a graphic dialog box in which the individual indicates his position and the intensity of pain symptoms experienced, following which this information is transferred to the computer. For example, should the individual activate a desk height adjustment, a dialog box can appear on the computer display screen, which initially asks whether there has been any change in the pain experienced by the individual and then shows the latest recorded entry. A suitable design variant may be to display a body image with predefined areas where the individual usually feels pain. The individual can than mark where the pain is felt and the intensity of the pain sensation. Where appropriate the individual can also append explanatory comments which can then be used in consultation with an occupational therapist.

The program can then either itself suggest a change in working position and if the system is designed to control furniture adjusting devices the adjustment can be made automatically, otherwise the individual himself is allowed to control the adjustment which is then registered by the relevant sensors.

As time goes on the computer gathers sensor data and pain records. The computer program is assumed to contain a self-learning analytical part, in the form of a neural network, for example. When sufficient data has been analysed, the program can begin to give warning of situations which the neural network, from experience, has found to be generally conducive to pain sensations. In the event of such warnings the individual can again be questioned about his pain condition, which gives the neural network the opportunity to further refine its warnings.

The computer preferably also comprises means of processing stored information and suggesting changes to any parameter on the basis of the processed information.

In a further refinement a window on the computer screen may be used to display a historical picture of sensor data and pain information over time, and a signal emitted which gradually becomes stronger and reminds the individual that it is now becoming more urgent to change working position.

## Claims

1. System for counteracting work-related stress injuries in an individual person when working in connection with at least one work-related object which permits adjustment or use of different working positions, the system comprising:
at least one sensor, provided at the object, for detecting the object's current adjustment or working position; and
a computer;
wherein the at least one sensor being arranged for delivering a signal containing information on the object's current adjustment or working position to the computer, in which the information is recorded and stored together with information on the current time, **characterised in that** the computer also comprises:
means for recording and storing information relating to pain symptoms experienced by the individual together with information on the current time, and
an analytical program linked to the recording arranged for collating and processing information in order to identify such combinations of sensor signals and other information, which have been shown to lead to pain sensations in the individual.

2. System according to Claim 1, **characterised in that** it also comprises a graphic dialogue box connected to the computer in which the individual marks the position and intensity of pain symptoms experienced, following which this information is transferred to the computer.

3. System according to either of the preceding Claims, **characterised in that** the object is an item of furniture.

4. System according to any one of the preceding Claims, **characterised in that** the object is a working tool.

5. System according to any one of the preceding Claims, **characterised in that** it also comprises means located on the individual for recording stresses and/or movement and for transferring this information, and storing and processing it on the computer

6. System according to any one of the preceding Claims, **characterised in that** at least one working tool used by the individual is provided with means of recording how the working tool is used and for transferring this information and storing and processing it on the computer.

7. System according to any one of the preceding Claims, **characterised in that** the computer comprises means of requesting the input of information from the individual when a given set parameter is modified.

8. System according to any one of the preceding Claims, **characterised in that** the computer also comprises means of processing stored information and suggesting changes to any parameter.

9. System according to any one of the preceding Claims, **characterised in that** the working tool is a computer with associated means of inputting and reading out information.

10. System according to Claim 9, **characterised in that** it comprises means of detecting activity for inputting information.

11. System according to either of Claims 9 or 10, **characterised in that** it comprises means of registering which program is being used on the computer that constitutes the working tool.

12. System according to any one of Claims 9 to 11, **characterised in that** one and the same computer is both working tool and computer for receiving, storing, processing and outputting system information.

13. System according to any one of the preceding Claims, **characterised in that** the system delivers warning information as soon as it identifies such combinations of sensor signals and other information that have been shown to lead to pain sensations in the individual.

14. System according to Claim 13, **characterised in that** it also comprises means of suggesting changes to working conditions.

15. System according to any one of the preceding Claims, **characterised in that** it also comprises means of displaying selected parts of sensor signals, pain indications and other information to the individual as a function of the time.

16. System according to any one of the preceding Claims, **characterised in that** it also comprises means of controlling adjusting mechanisms of the working tool(s).

## Patentansprüche

1. System, um arbeitsbedingten Verletzungen durch Beanspruchungen bei einer einzelnen Person entgegenzuwirken, wenn sie in Verbindung mit mindestens einem zu der Arbeit in Bezug stehenden Objekt arbeitet, das eine Einstellung oder die Verwendung unterschiedlicher Arbeitspositionen zulässt, wobei das System aufweist:
mindestens einen Sensor, der an dem Objekt vorgesehen ist, um die momentane Einstellung oder die Arbeitsposition des Objekts zu erfassen; und
einen Computer;
wobei der mindestens eine Sensor zum Zuführen eines Signals, das Informationen über die momentane Einstellung oder die Arbeitsposition des Objekts enthält, zu dem Computer, in dem die Informationen aufgezeichnet und zusammen mit Informationen über die momentane Zeit gespeichert werden, ausgelegt ist, **dadurch gekennzeichnet, dass** der Computer auch aufweist:
Einrichtungen zum Aufzeichnen und Speichern von Informationen, die sich auf Schmerz-Symptome beziehen, die durch die Person erfahren werden, zusammen mit Informationen über die momentane Zeit, und
ein analytisches Programm, das mit der Aufzeichnung verknüpft ist, das zum Sammeln und Verarbeiten von Informationen vorgesehen ist, um solche Kombinationen der Sensorsignale und anderer Informationen zu identifizieren, für die gezeigt worden ist, dass sie zu Schmerzempfindungen bei der Person führen.

2. System nach Anspruch 1, **dadurch gekennzeichnet, dass** es auch einen Grafik-Dialog-Kasten aufweist, der mit dem Computer verbunden ist, in dem die Person die Position und die Intensität von Schmerz-Symptomen, die sie erfahren hat, markiert, woraufhin diese Informationen zu dem Computer übertragen werden.

3. System nach irgendeinem der vorhergehenden Ansprüche, **dadurch gekennzeichnet, dass** das Objekt ein Möbelstück ist.

4. System nach einem der vorhergehenden Ansprüche, **dadurch gekennzeichnet, dass** das Objekt ein Arbeitsgerät ist.

5. System nach einem der vorhergehenden Ansprüche, **dadurch gekennzeichnet, dass** es auch Einrichtungen aufweist, die an der Person zum Aufzeichnen von Beanspruchungen und/oder einer Bewegung und zum Übertragen dieser Informationen zu und zum Speichern und Verarbeiten von diesen in dem Computer angeordnet sind.

6. System nach einem der vorhergehenden Ansprüche, **dadurch gekennzeichnet, dass** mindestens ein Arbeitsgerät, das durch die Person verwendet wird, mit Einrichtungen zum Aufzeichnen darüber, wie das Arbeitsgerät verwendet wird, und zum Übertragen dieser Informationen zu und zum Speichern und Verarbeiten von diesen in dem Computer versehen ist.

7. System nach einem der vorhergehenden Ansprüche, **dadurch gekennzeichnet, dass** der Computer Einrichtungen zum Anfordern der Eingabe von Informationen von der Person, wenn ein gegebener, eingestellter Parameter modifiziert ist, aufweist.

8. System nach einem der vorhergehenden Ansprüche, **dadurch gekennzeichnet, dass** der Computer auch Einrichtungen zum Verarbeiten gespeicherter Informationen und zum Vorschlagen von Änderungen irgendeines Parameters aufweist.

9. System nach einem der vorhergehenden Ansprüche, **dadurch gekennzeichnet, dass** das Arbeitsgerät einen Computer mit zugeordneten Einrichtungen zum Eingeben und Auslesen von Informationen ist.

10. System nach Anspruch 9, **dadurch gekennzeichnet, dass** es Einrichtungen zum Erfassen einer Aktivität zum Eingeben von Informationen aufweist.

11. System nach entweder Anspruch 9 oder 10, **dadurch gekennzeichnet, dass** es Einrichtungen zum Registrieren aufweist, welches Programm auf dem Computer verwendet wird, das das Arbeitsgerät bildet.

12. System nach einem der Ansprüche 9 bis 11, **dadurch gekennzeichnet, dass** ein und derselbe Computer sowohl das Arbeitsgerät als auch der Computer zum Aufnehmen, Speichern, Verarbeiten und Ausgeben von Systeminformationen ist.

13. System nach einem der vorhergehenden Ansprüche, **dadurch gekennzeichnet, dass** das System Warn-Informationen liefert, sobald es solche Kombinationen von Sensorsignalen und anderen Informationen identifiziert, für die gezeigt worden ist, dass sie zu Schmerzempfindungen bei der Person führen.

14. System nach Anspruch 13, **dadurch gekennzeichnet, dass** es auch Einrichtungen zum Vorschlagen von Änderungen der Arbeitsbedingungen aufweist.

15. System nach einem der vorhergehenden Ansprüche, **dadurch gekennzeichnet, dass** es auch Einrichtungen aufweist, um der Person ausgewählte Teile von Sensorsignalen, Schmerzanzeigen und anderen Informationen als eine Funktion der Zeit anzuzeigen.

16. System nach einem der vorhergehenden Ansprüche, **dadurch gekennzeichnet, dass** es auch Einrichtungen zum Steuern von Einstellmechanismen des Arbeitsgeräts (der Arbeitsgeräte) aufweist.

## Revendications

1. Système de lutte contre les fractures de sollicitation associées au travail chez un individu lorsqu'il travaille sur au moins un objet associé au travail qui permet un ajustement ou une utilisation de différentes positions de travail, le système comprenant :
au moins un capteur, prévu au niveau de l'objet, pour détecter l'ajustement ou la position de travail actuelle de l'objet ; et
un ordinateur ;
dans lequel le au moins un capteur est agencé pour délivrer un signal contenant des informations sur l'ajustement ou la position de travail actuelle de l'objet à l'ordinateur, dans lequel les informations sont enregistrées et stockées conjointement avec des informations sur l'heure actuelle, **caractérisé en ce que** l'ordinateur comprend également :
un moyen pour enregistrer et stocker des informations associées à des symptômes de douleur ressentis par l'individu conjointement avec des informations sur l'heure actuelle, et
un programme analytique lié à l'enregistrement agencé pour assembler et traiter des informations afin d'identifier des combinaisons de signaux de capteur et d'autres informations que l'on sait engendrer des sensations de douleur chez l'individu.

2. Système selon la revendication 1, **caractérisé en ce qu'**il comprend également une boite de dialogue graphique reliée à l'ordinateur dans laquelle l'individu indique la position et l'intensité des symptômes de douleur ressentis, à la suite de quoi ces informations sont transférées vers l'ordinateur.

3. Système selon l'une quelconque des revendications précédentes, **caractérisé en ce que** l'objet est un meuble.

4. Système selon l'une quelconque des revendications précédentes, **caractérisé en ce que** l'objet est un outil de travail.

5. Système selon l'une quelconque des revendications précédentes, **caractérisé en ce qu'**il comprend également un moyen situé sur l'individu pour enregistrer des sollicitations et/ou un mouvement et pour transférer ces informations et les stocker et les traiter sur l'ordinateur.

6. Système selon l'une quelconque des revendications précédentes, **caractérisé en ce qu'**au moins un outil de travail utilisé par l'individu est prévu avec un moyen d'enregistrement de la façon dont l'outil de travail est utilisé pour transférer ces informations et les stocker et les traiter sur l'ordinateur.

7. Système selon l'une quelconque des revendications précédentes, **caractérisé en ce que** l'ordinateur comprend un moyen de demande d'entrée d'informations de la part de l'individu lorsqu'un paramètre réglé donné est modifié.

8. Système selon l'une quelconque des revendications précédentes, **caractérisé en ce que** l'ordinateur comprend également un moyen de traitement d'informations stockées et de suggestion de changements d'un quelconque paramètre.

9. Système selon l'une quelconque des revendications précédentes, **caractérisé en ce que** l'outil de travail est un ordinateur muni de moyens associés d'entrée et de lecture d'informations.

10. Système selon la revendication 9, **caractérisé en ce qu'**il comprend un moyen de détection d'une activité pour entrer des informations.

11. Système selon l'une des revendications 9 ou 10, **caractérisé en ce qu'**il comprend un moyen de consignation du programme utilisé sur l'ordinateur qui constitue l'outil de travail.

12. Système selon l'une quelconque des revendications 9 à 11, **caractérisé en ce qu'**un seul et même ordinateur est à la fois un outil de travail et un ordinateur destiné à recevoir stocker traiter et sortir des informations sur le système.

13. Système selon l'une quelconque des revendications précédentes, **caractérisé en ce que** le système délivre des informations d'avertissement dés qu'il identifie des combinaisons de signaux de capteur et d'autres informations que l'on sait engendrer des sensations de douleur chez l'individu.

14. Système selon la revendication 13, **caractérisé en ce qu'**il comprend également un moyen de suggestion de changements de condition de travail.

15. Système selon l'une quelconque des revendications précédentes, **caractérisé en ce qu'**il comprend également un moyen d'affichage de parties sélectionnées de signaux de capteurs, d'indications de douleur et d'autres informations pour l'individu en fonction de l'heure.

16. Système selon l'une quelconque des revendications précédentes, **caractérisé en ce qu'**il comprend également un moyen de contrôle de mécanismes d'ajustement du/des outil(s) de travail.
